# EUROPÄISCHE PATENTSCHRIFT

(11) **EP 1 526 870 B1**
(45) Veröffentlichungstag und Bekanntmachung des Hinweises auf die Patenterteilung: **02.05.2007**
(21) Anmeldenummer: 03790851.4
(22) Anmeldetag: 04.08.2003
(51) Int. Cl.: A61K 31/56, A61K 31/404, A61P 37/00, A61P 11/06

(54) **KOMBINATION VON LOTEPREDNOLETABONAT UND DFHO ZUR BEHANDLUNG VON ATEMWEGSERKRANKUNGEN, ALLERGISCHEN ERKRANKUNGEN, ASTHMA UND COPD**
COMBINATION OF LOTEPREDNOLETABONAT AND DFHO FOR TREATING RESPIRATORY DISEASES, ALLERGIC DISEASES, ASTHMA AND COPD
COMBINAISON DE LOTEPREDNOLETABONAT ET DFHO POUR TRAITER DES MALADIES DES VOIES RESPIRATOIRES, DES MALADIES ALLERGIQUES, DE L'ASTHME ET DES MALADIES PULMONAIRES CHRONIQUES OBSTRUCTIVES

(30) Priorität: 09.08.2002 DE 10236688
(43) Veröffentlichungstag der Anmeldung: 04.05.2005
(73) Patentinhaber: MEDA Pharma GmbH & Co. KG, 61352 Bad Homburg (DE)
(72) Erfinder: LOCHER, Mathias, 63549 Ronneburg (DE); HERMANN, Robert, 63452 Hanau (DE)
(86) Internationale Anmeldenummer: PCT/EP2003/008607
(87) Internationale Veröffentlichungsnummer: WO 2004/019984

(56) Entgegenhaltungen:
- WO-A-03/039548
- DE-A- 19 917 504
- GB-A- 2 079 755
- US-A- 4 996 335
- US-B1- 6 251 923
- HAMMERBECK D M ET AL: "Effects of several glucocorticosteroids and PDE4 inhibitors on increases in total lung eosinophil peroxidase (EPO) levels following either systemic or intratracheal administration in sephadex- or ovalbumin-induced inflammatory models." INFLAMMATION. UNITED STATES AUG 2000, Bd. 24, Nr. 4, August 2000 (2000-08), Seiten 317-329, XP009020362 ISSN: 0360-3997
- COMPTON C H ET AL: "Cilomilast, a selective phosphodiesterase-4 inhibitor for treatment of patients with chronic obstructive pulmonary disease: a randomised, dose-ranging study" LANCET, XX, XX, Bd. 358, Nr. 9278, 28. Juli 2001 (2001-07-28), Seiten 265-270, XP004297349 ISSN: 0140-6736
- WHITTAKER A J ET AL: "Inhaled steroid therapy in chronic obstructive pulmonary disease." CURRENT OPINION IN PULMONARY MEDICINE. UNITED STATES MAR 2000, Bd. 6, Nr. 2, März 2000 (2000-03), Seiten 104-109, XP009020341 ISSN: 1070-5287 in der Anmeldung erwähnt
- TORPHY T J ET AL: "Ariflo (SB 207499), a second generation phosphodiesterase 4 inhibitor for the treatment of asthma and COPD: from concept to clinic." PULMONARY PHARMACOLOGY & THERAPEUTICS. ENGLAND 1999, Bd. 12, Nr. 2, 1999, Seiten 131-135, XP001155797 ISSN: 1094-5539
- BARNES P J: "Chronic obstructive pulmonary disease: new opportunities for drug development" TRENDS IN PHARMACOLOGICAL SCIENCES, ELSEVIER TRENDS JOURNAL, CAMBRIDGE, GB, Bd. 19, Nr. 10, Oktober 1998 (1998-10), Seiten 415-423, XP004156947 ISSN: 0165-6147

## Beschreibung

Die vorliegende Erfindung betrifft eine neue Kombination von dem Glucocorticoid Loteprednol, und dem Phosphodiesterase-4 Inhibitor (PDE-4-Inhibitor) N-(3,5-Dichlorpyridin-4-yl)-2-[1-(4-fluorbenzyl)-5-hydroxyindol-3-yl]-2-oxoacetamid) (DFHO), für eine simultane, sequenzielle oder separate Verabreichung bei der Behandlung von Atemwegserkrankungen, allergischen Erkrankungen, Asthma und chronisch obstruktiven Lungenerkrankungen (COPD).

Allergischen Erkrankungen und chronisch obstruktiven Lungenerkrankungen (chronic obstructive pulmonary diseases, COPD) liegen entzündliche Prozesse zugrunde, die durch eine erhöhte Anzahl an Entzündungszellen und erhöhter Freisetzung resp. Sekretion von Entzündungsmediatoren gekennzeichnet sind. Studien über die letzten 20 Jahre haben ergeben, dass eine Entzündung der Atemwege für die Atemweg-Fehlfunktion bei Asthma und COPD von zentraler Bedeutung ist. Vergleichbare Veränderungen wurden bei allergischen Entzündungen der Nase und der Augen beobachtet. Üblicherweise wird die Schleimhaut durch eine Vielzahl von Zellen infiltriert, einschließlich Mastzellen, Eosinophilen und Lymphozyten. Diese Zellen setzen eine Reihe von Mediatoren frei, zu denen insbesondere Interleukin-4 (IL-4), GM-CSF (Granulocyte/Macrophage colony-stimulating factor) und der Tumomekrose Faktor α (TNF-α) gehören, die letztendlich die Entzündungen und die Symptome der allergischen Erkrankungen und der COPD bewirken.
Zum gegenwärtigen Zeitpunkt wird bei allen allergischen Erkrankungen ein ähnlicher entzündungshemmender Therapieansatz verfolgt. Die Pathologie dieser Erkrankungen ergab, dass der Entzündungsprozess in der Schleimhaut von Patienten primär die Symptomaktivität bestimmt. Unter den entzündungshemmenden Verbindungen, die zur Zeit zur Behandlung von Asthma, Rhinitis oder Konjunktivitis bereitstehen, sind die Glucocorticoide die wirksamsten. Bevorzugt werden topisch verabreichbare Wirkstoffe über eine inhalative, intranasale oder intraokuläre Verabreichung eingesetzt. Aufgrund der erfolgreichen Verwendung von inhalierbaren Glucocorticoiden bei der Behandlung und Prävention von Atemwegsentzündungen und bleibenden Lungenschäden bei Asthmapatienten wurde dieser therapeutische Ansatz auch auf COPD-Patienten übertragen, obwohl keine Daten vorliegen, die eine Langzeitwirksamkeit dieser Wirkstoffe bei COPD-Patienten eindeutig belegen könnten (Whittaker AJ, Spiro SG; Curr Opin Pum Med 2000; 6:104-9).

Eine der wichtigsten entzündungshemmenden Eigenschaften von Glucocorticoiden liegt in der Inhibierung der Cytokinfreisetzung. Es ist bekannt, dass mehrere Cytokine wie IL-4, IL-5, GM-CSF und TNF- α an der Atemwegsentzündung beteiligt sind. Die Wirksamkeit von Glucocorticoiden kann teilweise über die inhibierende Wirkung auf die Cytokinsynthese und Cytokinfreisetzung erklärt werden (Marx et al.; Pulm Pharmacol Ther 2002; 15:7-15).

Ein Nachteil von Glucocorticoiden liegt in ihren möglichen systemischen Nebenwirkungen wie beispielsweise Wachstumsverlangsamung oder auch Osteoporose. Sinnvolle Maßnahmen zur Verminderung des Risikos von Nebenwirkungen bei topischer Applikation von Glucocorticoiden stellen die Verwendung der minimal wirksamen Dosis oder die Einschränkung der systemischen Verfügbarkeit des Wirkstoffs dar. Einen neuen Weg eröffnet die Verwendung von sogenannten "Soft-Steroiden". Im Gegensatz zu anderen Glucocorticoiden, die meistens erst in der Leber zu pharmakodynamisch inaktiven Metaboliten abgebaut werden, erfolgt bei den "Soft-Steroiden" die metabolische Inaktivierung zum Teil bereits an der Stelle ihrer Verabreichung (intranasal, okulär oder intrapulmonal). In Folge dieser partiellen lokalen Metabolisierung gelangt keine oder nur sehr wenig pharmakodynamisch aktive Substanz in den systemischen Blutkreislauf, so dass praktisch mit den steroidspezfischen Nebenwirkungen nicht zu rechnen ist. Das prominenteste Beispiel dieser neuen Wirkstoffklasse stellt Loteprednol dar, das für die Therapie der allergischen Konjunktivitis und Uveitis bereits zugelassen ist.

Eine weitere Klasse von potentiellen Therapeutika bei allergischen Erkrankungen und COPD stellen die Phosphodiesterase-4 Inhibitoren dar. Phosphodiesterase-Enzyme sind für die Inaktivierung von cyclischem Adenosinmonophosphat (cAMP) und cyclischem Guanosinmonophosphat (cGMP) verantwortlich. Eine Inhibierung der Phosphodiesterase-4 führt zu einer Erhöhung von cAMP in den Zellen, was wiederum zu einer Herunterregulierung der Funktion von nahezu allen proinflammatorischen Zellen respektive Immunzellen führt. Interessanterweise exprimieren Entzündungszellen, die an der Pathogenese von Erkrankungen wie Asthma, Konjunktivitis, Rhinitis oder chronisch obstruktiver Lungenerkrankung beteiligt sein sollen, bevorzugt die Phosphodiesterase-4-Enzyme.

In den letzten Jahren wurde die Entwicklung von Phosphodiesterase-4 Inhibitoren vorangetrieben, die zur Therapie von allergischen Erkrankungen, Asthma oder COPD einsetzbar sind. Die in vitro inhibitorische Aktivität auf die Cytokinfreisetzung und die therapeutische Effizienz in Asthma-Modellen konnten beispielsweise für die Wirkstoffe Roflumilast, Cilomilast oder auch Piclamilast gezeigt werden (Torphy et al.; Pulm Pharmacol Ther 1999; 12:131-5; Poppe et al.; Allergy 2000; 55(Suppl 63):270; Giembycz MA; Expert Opin Investig Drugs 2001; 10:1361-79; Ezeamuzie CI; Eur J Pharmacol 2001; 417:11-8). Von besonderem Interesse ist eine neue Klasse von substituierten Hydroxyindolen, die in der DE 19 818 964, der DE 19 917 504 und der US 6,251,923 beschrieben sind, sowie auch neue 7-Azaindole, die in der DE 10 053 275 und der PCT/EP 01/12376 offenbart sind.

Überraschenderweise wurde nun gefunden, dass die neue Kombination von dem Glucocorticoid Loteprednol mit dem Phosphodiesterase-4 Inhibitor N-(3,5-Dichlorpyridin-4-yl)-2-[1-(4-fluorbene)-5-hydroxyindol-3-yl]-2-oxoacetamid) (DFHO) bei der Behandlung von Atemwegserkrankungen, allergischen Erkrankungen, Asthma und/oder chronisch obstruktiven Lungenerkrankungen vorteilhaft ist. Die add-on Therapie von dem Phosphodiesterase-4 Inhibitor N-(3,5-Dichlorpyridin-4-yl)-2-[1-(4-fluorbenzyl)-5-hydroxyindol-3-yl]-2-oxoacetamid, der oral, intranasal oder inhalativ verabreicht werden kann, mit dem topischen Glucocorticoid Loteprednol zeichnet sich durch eine verbesserte therapeutische Wirksamkeit aus, wie auch durch Auftreten von weniger Nebenwirkungen.

Die Erfindung dient der Verbesserung der Therapie bei Atemwegserkrankungen, allergischen Erkrankungen, Asthma und chronisch obstruktiven Lungenerkrankungen, wie auch deren Prophylaxe. Mit dem in der Kombination enthaltenen Phosphodiesterase-4 Inhibitor DFHO und dem Glucocorticoid Loteprenol können die den Krankheitsbildern zugrundeliegenden Entzündungen erfolgreich bekämpft werden. Zudem führt die add-on Therapie mit dem Phosphodiesterase-4 Inhibitor zu einem geringeren Verbrauch an dem Glucocorticoid, was das Risiko von Nebenwirkungen vermindert.
Die vorliegende Erfindung betrifft daher die Verwendung einer Kombination, die das Glucocorticoid Loteprednol und den Phosphodiesterase-4 Inhibitor DFHO in fixer oder freier Kombination umfasst, zur Herstellung eines Arzneimittels zur Behandlung von Atemwegserkrankungen, allergischen Erkrankungen, Asthma und/oder chronisch obstruktiven Lungenerkrankungen.

Gemäß einer bevorzugten Ausführungsform der Erfindung wird ein pharmazeutisch annehmbarer Ester des Loteprednol, insbesondere Loteprednoletabonat, verwendet. Die Herstellung von Loteprednol und Loteprednoletabonat ist beispielsweise in dem deutschen Patent DE 3 126 732, dem korrespondieren US-Patent 4,996,335 und dem korrespondierenden japanischen Patent JP-89011037 beschrieben.

Der im Rahmen der vorliegenden Erfindung eingesetzte Phosphodiesterase-4 Inhibitor N-(3,5-Dichlorpyridin-4-yl)-2-[1-(4-fluorbenzyl)-5-hydroxyindol-3-yl]-2-oxoacetamid (im Folgenden "DFHO"), ist beispielsweise in der DE 19 818 964 beschrieben. Er kann auch als pharmazeutisch verträgliches Salz eingesetzt werden, wie sie dem Fachmann bekannt sind.

Die erfindungsgemäße Kombination von Loteprednoleatbonat mit DFHO kann sowohl prophylaktisch wie auch nach dem Auftreten von Symptomen verabreicht werden. Sie kann auch dazu dienen, das Fortschreiten der Krankheiten zu verlangsamen oder zu verhindern.

Die nachfolgende Versuchsbeschreibung dient der näheren Erläuterung der erfindungsgemäßen Lehre.

### Hemmung der GM-CSF-Freisetzung aus LPS-stimulierten Monozyten

EDTAisiertes humanes Vollblut wurde mit Hanks-Puffer im Verhältnis 1:1 gemischt. Histopaque-1077 Lösung (15ml) wurde mit max, 40ml der Blut:Hanks-Mischung vorsichtig überschichtet und für 30 min bei Raumtemperatur abzentrifugiert (2000UpM). Die mit Leukozyten angereicherte Bande wurde abgezogen, zweimal mit Hanks-Puffer gewaschen und in RPMI 1640 Medium mit Glutamax I (Gibco BRL, Eggenstein) transferiert. Die Abtrennung der Monozyten erfolgte durch deren Adhärenz an die Zellkulturflasche über einen Zeitraum von zwei Stunden. Anschließend wurden die Zellen gründlich mit Medium gewaschen, um nicht adhärente Zellen zu entfernen. Die erhaltenen Monozyten wurden in RPMI 1640 Medium mit 10% hitzeinaktiviertern fötalern Kälberserum (FKS) und 100U/ml Penicillin und 100µg/ml Streptomycin im CO₂-Inkubator (5% CO₂, 96% relative Luftfeuchtigkeit, 37°C) kultiviert.
Primäre Monozyten wurden in 24-Loch-Platten mit 5×10⁵ Zellen/Loch ausgesät. Die Zellen wurden mit den aufgeführten Testsubstanzen für 30 Minuten präinkubiert. Danach wurde LPS zugegeben und für einen weiteren Zeitraum von 24h inkubiert. Die Überstände wurden abgezogen und mittels ELISA untersucht.
Zur Bestimmung der Menge an sezerniertem humanen GM-CSF in den Zellkultur-Überständen wurde ein OptEIA^{™} Human GM-CSF ELISA-Test (Pharmingen, San Diego) angewendet. Die Durchführung erfolgte in Mikrotiterplatten. Als Antikörper wurden antihumane monoklonale Antikörper über Nacht bei 4°C an die Platte gekoppelt. Nach dieser Beschichtung erfolgte nach dreimaligem Waschen ein Absättigen unspezifischer Bindungen mittels Assay-Diluent-Lösung^{™} (PBS mit 10% FKS, pH 7,0) (Pharmingen, San Diego) bei RT für 1h. Anschließend wurde mit den Proben und dem Standard (humanes rekombinantes GM-CSF) über Nacht bei 4°C inkubiert. Die Proben wurden unverdünnt oder in einer Verdünnung 1:50, der Standardverdünnungen gemäß Protokoll ausgehend von einer Stock-Lösung mit 500 pg/ml humanen GM-CSF, hergestellt. Der Nachweis von gebundenem humanen GM-CSF erfolgte mit Hilfe von monoklonalen biotinylierten anti-human GM-CSF-Antikörpern und einem Avidin-Meerrettich-Peroxidase-Reagenz für 1h bei RT. Nach allen Schritten erfolgte fünf- bzw. siebenmaliges Waschen mit PBS/0,05% Tween 20. Die Enzymaktivität wurde mit Substrate Solution^{™} (Tetramethylbenzidin (TMB) und Wasserstoffperoxid, Pharmingen, San Diego) als Substrat für 30 min bei RT bestimmt. Die Enzym-Substratreaktion wurde mit 1M Phosphorsäure abgestoppt und die Extinktion bei 450nm gemessen.

### Ergebnisse

Zunächst wurden Dosis-Wirkungskurven für N-(3,5-Dichlorpyridin-4-yl)-2-[1-(4-fluorbenzyl)-5-hydroxyindol-3-yl]-2-oxoacetamid (DFHO) und Loteprednol separat erstellt. Aus diesen wurde jeweils der IC₅₀-Wert für die GM-CSF-Freisetzung aus humanen Monozyten für DFHO von 3,2 µM und für Loteprednol von 53,7 nM errechnet. In weiteren Versuchen wurden IC₅₀-Werte für DFHO und Loteprednol in Gegenwart von sub-IC₅₀-Konzentrationen der jeweils anderen Substanz erstellt. Dabei emiedrigte die Zugabe von 5nM DFHO den IC₅₀-Wert für Loteprednol von 53,7nM auf 13,4nM. Umgekehrt emiedrigte die Zugabe von 10 nM Loteprednol den IC₅₀-Wert für DFHO von 3,2µM auf 0,06µM,
Die ermittelten IC₅₀-Werte für Loteprednol für die TNF- bzw. GM-CSF-Freisetzung aus LPS-stimulierten Monozyten entsprechen denen in der Literatur angegebenen IC₅₀-Werten für andere Zellsysteme. Das bedeutet, daß das verwendete Zellsystem valide und geeignet ist, und die für das Projektziel nötigen Untersuchungen mit diesem System zu verläßlichen Aussagen kommen. Die IC₅₀-Werte für DFHO entsprechen denen in der Patentliteratur angegebenen Werten.
Bei der Gabe von 5nM DFHO emiedrigte sich der IC₅₀-Wert für Loteprednol auf die TNF-Freisetzung um 65 % und für die GM-CSF-Freisetzung um 75 %. Die Konzentration von 5 nM DFHO liegt weit unter dem IC₅₀-Wert für diese Substanz von 5,7 µM bzw. 3,2 µM, so daß allein gegeben, keine Wirkung von 5 nM DFHO zu beobachten ist.
Umgekehrt erniedrigte sich der IC₅₀-Wert für DFHO auf die TNF-Freisetzung um 99 % und für die GM-CSF-Freisetzung um 98 % bei der gleichzeitigen Gabe von 10 nM Loteprednol. Die Konzentration von 10 nM Loteprednol liegt weit unter dem IC₅₀-Wert für diese Substanz von 85,5 nM bzw. 53,7 nM, so daß allein gegeben, keine Wirkung von 10 nM Loteprednol zu beobachten ist.
Überraschend und für den Fachmann nicht vorhersehbar ist hier ein überadditiver Effekt bei der Hemmung der TNF- und GM-CSF-Freisetzung zu beobachten, der durch die gleichzeitige Verabreichung von Loteprednol und DFHO hervorgerufen wird.

Zur Verabreichung der erfindungsgemäßen Kombination der Wirkstoffe sind die im Folgenden erwähnten Darreichungsformen besonders geeignet.

So können die in der Kombination enthaltenen Wirkstoffe beispielsweise separat als zwei orale Formulierungen verabreicht werden, oder ein Wirkstoff liegt als orale Formulierung vor, und der andere in topischer Form (intranasal, inhalativ).
Gemäß einer Ausführungsform der Erfindung kann der Phosphodiesterase-4 Inhibitor oral verabreicht werden. Zur Anwendung kommen dabei übliche galenische Zubereitungsformen wie Tabletten, Sirup, Kapseln, Präparationen mit verlangsamter Freisetzung (Retardformulierung), Pastillen oder Brausegranulate.

Feste Arzneiformen wie Tabletten können inerte Inhalts- und Trägerstoffe enthalten, wie beispielsweise Calciumcarbonat, Calciumphosphat, Natriumphosphat, Lactose, Stärke, Mannit, Alginate, Gelatine, Guar-Gummi, Magnesium- oder Aluminiumstearat, Methylcellulose, Talkum, hochdisperse Kieselsäuren, Silikonöl, höhermolekulare Fettsäuren (wie Stearinsäure), Agar-Agar oder pflanzliche oder tierische Fette und Öle, feste hochmolekulare Polymere (wie Polyethylenglycol); für orale Applikationen geeignete Zubereitungen können gegebenenfalls zusätzliche Geschmacks- oder Süßstoffe enthalten. Die Zusammensetzungen in Kapselform können nach allgemein üblichen Verfahren hergestellt werden, beispielsweise durch Verwendung der vorstehend genannten Trägerstoffe in einer Hartgelatine-Kapselschale. Für Zusammensetzungen in Form von Weichgelatine-Kapseln können üblicherweise zur Herstellung von Dispersionen oder Suspensionen verwendete pharmazeutische Träger eingesetzt werden, wie beispielsweise wässrige Gummis, Cellulosen, Silicate oder Öle, die in eine Weichgelatine-Kapselschale eingebaut werden. Sirupformulierungen bestehen üblicherweise aus einer Suspension oder Lösung der Verbindung oder eines Salzes davon in einem flüssigen Träger wie beispielsweise Ethanol, Erdnussöl, Olivenöl, Glycerin oder Wasser, wobei Geschmacks- und Farbstoffe enthalten sein können.
Durch die topische Verabreichung der erfindungsgemäßen Kombination können therapeutisch wirksame Konzentrationen bereits bei niedrigen Dosierungen erreicht werden. Deshalb werden im Rahmen der vorliegenden Erfindung topische Zubereitungsformen bevorzugt, zu denen insbesondere intranasale und inhalative Formulierungen gehören. Intranasale Zubereitungen können als wässrige oder ölige Lösungen, Suspensionen oder Emulsionen verabreicht werden. Für die inhalative Verabreichung eines Wirkstoffes kann dieser in Form einer Suspension, Lösung oder Emulsion verabreicht werden, die als trockenes Pulver oder als Aerosol vorliegt, wobei alle üblichen Treibmittel verwendet werden können.
Gemäß einer bevorzugten Ausführungsform der Erfindung liegt die Phosphodiesterase-4 Inhibitor-Zusammensetzung in Form eines Nasensprays oder eines Dosieraerosols oder eines Dosier-Trockenpulvers zur Inhalation vor. Die Glucocorlicoid-Zusammensetzung ist bevorzugt ebenfalls eine topische Präparation mit einer bevorzugten Formulierung in Form von Nasenspray, Dosieraerosol oder Dosier-Trockenpulver zur Inhalation.
Das erfindungsgemäß eingesetzte Soft-Steroid Loteprednoletabonat wird bevorzugt als Suspension in Wasser formuliert, mit weiteren Bestandteilen wie Konservierungsstoffen, Stabilisatoren, Isotonierungsmitteln, Verdickungsmitteln, Suspensionsstabilisatoren, Hilfsstoffen zur pH-Wert-Einstellung, Puffersystemen und Netzmitteln. Für weitere Details bezüglich geeigneter Hilfsstoffe wird beispielsweise auf die DE 19 947 234 verwiesen.

Die erfindungsgemäßen pharmazeutischen Zubereitungen können neben den wirksamen Bestandteilen Loteprednol und DFHO weitere Bestandteile wie übliche Konservierungsstoffe, Stabilisatoren, Verdickungsmittel, Geschmacksstoffe etc. enthalten.

### Ausführungsbelspiel

Nasenspraysuspension mit Loteprednoletabonat (1%)

| | |
|---|---|
| Loteprednoletabonat | 1,000 g |
| Avicel RC 591 | 1,100 g |
| Polysorbat 80 | 0,100 g |
| Sorbitollösung 70% | 6,000 g |
| Natriumedetat | 0,050 g |
| Benzalkoniumchlorid | 0,020 g |
| Gereinigtes Wasser | ad 100 ml |

### Herstellung

In einem geeigneten Rührwerksbehälter mit Homogenisiereinrichtung 45 kg gereinigtes Wasser voriegen und darin Avicei RC 591 hochtourig einhomogenisieren. Danach miteinan-der die Stoffe Polysorbat 80, Sorbitollösung, Natriumedetat und Benzalkoniumchlorid unter Rühren auflösen.
Anschließend den Wirkstoff Loteprednoletabonat hochtourig einhomogenisieren, bis eine gleichmäßige Suspension entstanden ist. Danach auf das Endvolumen mit gereinigtem Wasser auffüllen und weiter homogenisieren. Anschließend die Suspension evakuieren um die entstandenen Luftblasen zu entfernen. Die entstandene Suspension wird anschließend in Flaschen abgefüllt, welche danach mir einer geeigneten Nasenspraypumpe versehen werden.

In einer vorteilhaften Ausführungsform liegen die wirksamen Komponenten dieser Kombination in Form einer fixen Kombination vor, wodurch die Anwendung für den Patienten vereinfacht ist. Die Verabreichung der Wirkstoffe kann dabei simultan, sequenziell oder separat in freier oder fixer Kombination erfolgen. Sie können sowohl in einer Einzeldosisform verabreicht werden, wie auch als zwei getrennte Formulierungen, die gleich oder verschieden sein können. Die Zugabe kann zur gleichen Zeit, d. h. simultan, erfolgen, oder zeitlich getrennt, worunter sowohl kurze als auch lange Abstände zu verstehen sind, wie beispielsweise die Verabreichung von Loteprednol abends und die Verabreichung von DFHO morgens, oder auch umgekehrt.

Die Wirkstoffe können von 1 bis 6 mal täglich verabreicht werden. Bevorzugt werden die Wirkstoffe 1 bis 2 mal täglich verabreicht, besonders bevorzugt 2 mal täglich. Die Dosis von DFHO beträgt ungefähr 0,1 bis 20 mg pro Tag pro Erwachsener, bevorzugt zwischen 0,2 und 5 mg. Die Dosis von Loteprednol kann im Bereich der zugelassenen Dosierung liegen, d. h. im Rahmen von 0,1 bis 1,6 mg pro Tag, bevorzugt zwischen 0,2 und 0,8 mg pro Tag. Die tatsächliche Dosis hängt vom allgemeinen Zustand der Patienten (Alter, Gewicht, etc. ) und dem Schweregrad der Erkrankung ab.

## Patentansprüche

1. Verwendung der fixen oder freien Kombination von Loteprednoletabonat und N-(3,5-Dichlorpyridin-4-yl)-2-[1-(4-fluorbenzyl)-5-hydroxyindol-3-yl]-2-oxoacetamid (DFHO) oder seiner pharmazeutisch verträglichen Salze zur Herstellung eines Arzneimittels zur Behandlung und Prophylaxe von Atemwegserkrankungen, allergischen Erkrankungen, Asthma und/oder chronisch obstruktiven Lungenerkrankungen.

## Claims

1. The use of the fixed or free combination of loteprednol etabonate and N-(3,5-dichloropyridin-4-yl)-2-[1-(4-fluorobenzyl)-5-hydroxyindol-3-yl]-2-oxoacetamide (DFHO) or its pharmaceutically acceptable salts for producing a medicament for the treatment and prophylaxis of respiratory diseases, allergic diseases, asthma and/or chronic obstructive pulmonary diseases.

## Revendications

1. Utilisation de la combinaison fixe ou libre d'étabonate de lotéprednol et de N-(3,5-dichloropyridin-4-yl)-2-[1-(4-fluorobenzyl)-5-hydroxyindol-3-yl]-2-oxo-acétamide) (DFHO) ou de ses sels acceptables d'un point de vue pharmaceutique pour préparer un médicament destiné au traitement et à la prophylaxie des maladies des voies respiratoires, des maladies allergiques, de l'asthme et/ou des broncho-pneumopathies chroniques obstructives.
